# EUROPÄISCHE PATENTSCHRIFT

(11) **EP 4 601 586 B1**
(45) Veröffentlichungstag und Bekanntmachung des Hinweises auf die Patenterteilung: **10.12.2025**
(21) Anmeldenummer: 23789525.5
(22) Anmeldetag: 09.10.2023
(51) Int. Cl.: A61F 2/64, A61F 2/68, A61F 2/74, A61F 2/50

(54) **GELENK**
JOINT
ARTICULATION

(30) Priorität: 10.10.2022 DE 102022126219
(43) Veröffentlichungstag der Anmeldung: 20.08.2025
(73) Patentinhaber: Otto Bock Healthcare Products GmbH, 1110 Wien (AT)
(72) Erfinder: BOHLAND, Andreas, 1110 Wien (AT); PICKERILL, Thomas West, 1110 Wien (AT); SIEBERT, Michael, 1110 Wien (AT); SCHUH, Andreas, 1110 Wien (AT)
(74) Vertreter: Gramm, Lins & Partner Patent- und Rechtsanwälte PartGmbB
(86) Internationale Anmeldenummer: PCT/EP2023/077907
(87) Internationale Veröffentlichungsnummer: WO 2024/079053

(56) Entgegenhaltungen:
- EP-A1- 3 522 835
- US-B2- 11 213 407

## Beschreibung

Die Erfindung betrifft ein Gelenk für eine orthopädietechnische Einrichtung, wobei das Gelenk ein erstes Gelenkteil, ein zweites Gelenkteil, das um eine Schwenkachse schwenkbar an dem ersten Gelenkteil angeordnet ist, und ein Hydrauliksystem aufweist, dass eine erste Hydraulikkammer mit einer ersten Fluidleitung und eine zweite Hydraulikkammer mit einer zweiten Fluidleitung aufweist, wobei die beiden Fluidleitungen miteinander verbunden sind, wobei beim Verschwenken des ersten Gelenkteils relativ zum zweiten Gelenkteil Hydraulikfluid aus einer Hydraulikkammer in die andere Hydraulikkammer geleitet wird.

Derartige Gelenke sind aus dem Stand der Technik seit langem bekannt und werden beispielsweise für Orthesen oder Prothesen als Gelenk verwendet. Werden die beiden Gelenkteil relativ zueinander um die Schwenkachse verschwenkt, wird Hydraulikfluid aus einer der Hydraulikkammern in die jeweils andere Hydraulikkammer geleitet. Dies kann aktiv geschehen, in dem das Fluid zwischen den Kammern hin und her gepumpt wird. **In** diesem Fall wird das Fluid verwendet, um das Gelenk zu bewegen, also die beiden Gelenkteile relativ zueinander zu verschwenken. Alternativ oder zusätzlich dazu kann das Verschwenken der beiden Gelenkteile relativ zueinander auch auf andere Weise hervorgerufen werden. Unabhängig davon stellen die Fluidleitungen dem strömenden Fluid einen Strömungswiderstand entgegen. Die Größe dieses Strömungswiderstandes bestimmt unter anderem, wie schnell das Fluid von einer Kammer in die andere gelangt und damit, wie schnell die beiden Gelenkteile bei vorgegebener Kraft relativ zueinander verschränkt werden. Der Strömungswiderstand ist folglich ein Maß für die Dämpfung der Bewegung des Gelenks.

In vielen Ausführungsformen befinden sich die beiden Hydraulikkammern in einem einzigen Gehäuse, beispielsweise einem Zylinder, und sind durch einen Kolben, der beide Hydraulikkammern räumlich begrenzt, voneinander getrennt. Wird nun das Gelenk bewegt, also das erste Gelenkteil relativ zum zweiten Gelenkteil verschwenkt, wird der Kolben innerhalb des Zylinders bewegt und somit Hydraulikfluid aus einer der Hydraulikkammern herausgedrückt. Über die Fluidleitungen gelangt dieses Fluid in die andere Hydraulikkammer.

Wird der Kolben in dieser Ausgestaltung durch eine Kolbenstange geführt, die sich beispielsweise nur durch eine der beiden Hydraulikkammern erstreckt, nimmt das in den beiden Hydraulikkammern für das Hydraulikfluid zur Verfügung stehende Volumen mit einer Bewegung des Kolbens und der daran angeordneten Kolbenstange zu oder ab. Das Volumen nimmt ab, wenn die Hydraulikkammer, in der sich die Kolbenstange nicht erstreckt, verkleinert wird und das Volumen nimmt zu, wenn diese Hydraulikkammer durch die Bewegung des Kolbens vergrößert wird.

Da ein Hydraulikfluid in der Regel inkompressibel ist, kann diese Volumenänderung, die mit der Bewegung des Gelenkes einhergeht, nicht durch eine Kompression oder Expansion des Hydraulikfluids aufgefangen werden. Es ist daher ein Ausgleichsvolumen vorhanden, das überschüssiges Fluid aufnimmt oder fehlendes Fluid bereitstellt. Ein solches Ausgleichsvolumen ist auch für den Fall sinnvoll, dass aufgrund thermischer Ausdehnung das Volumen des im Hydrauliksystem enthaltenen Hydraulikfluids zu- oder abnimmt und wird daher regelmäßig auch dann benötig, wenn sich die Kolbenstange durch beide Hydraulikkammern erstreckt.

Ein solches System ist beispielsweise aus der US 11,213,407 B2 bekannt. Es ist wichtig, dass bei einer Bewegung des Gelenkes das Ausgleichsvolumen mit der Hydraulikkammer und der Fluidleitung verbunden ist, die den geringeren Druck aufweist. Damit wird verhindert, dass die Bewegung des Gelenkes dazu führt, zu viel Hydraulikfluid in das Ausgleichsvolumen zuleiten. Im Stand der Technik wird dies durch eine recht komplizierte und aufwändige Anordnung von mindestens vier Rückschlagventilen erreicht. Durch die komplizierte Ausgestaltung ist ein solches Gelenk aufwändig und damit kostenintensiv in der Herstellung und benötigt zudem relativ viel Platz. Andere Ausgestaltungen verwenden zwei steuerbare Ventile, von den jeweils eines das Ausgleichsvolumen mit jeder der beiden Fluidleitungen verbindet. Dadurch ist jedoch ein relativ hoher Steuerungsaufwand nötig und auch die Anzahl und Komplexität der benötigten Teile ist hoch, da beispielsweise oft zusätzlich zu den komplexen steuerbaren Ventilen Rückschlagventile benötig werden.

Eine andere Ausgestaltung eines Gelenks für eine orthopädietechnische Einrichtung, insbesondere für ein Kniegelenk, ist aus der WO 2018/065615 A1 bekannt. Auch dieses Gelenk weist ein Hydrauliksystem auf, das verwendet wird, um Widerstände, die einer Bewegung des Gelenkes entgegengesetzt werden, einzustellen. Dies ist für unterschiedliche Bewegungsrichtungen unterschiedlich möglich.

Der Erfindung liegt daher die Aufgabe zugrunde, ein Gelenk gemäß dem Oberbegriff des Anspruchs eins so weiterzuentwickeln, dass es kostengünstig und klein herstellbar ist.

Die Erfindung löst die gestellte Aufgabe durch ein Gelenk der oben beschriebenen Art, das sich dadurch auszeichnet, dass die Fluidleitungen durch ein Drosselventil und ein 3-Wege-Ventil verbunden sind, die parallel zueinander wirken, wobei der dritte Ausgang des 3-Wege-Ventils mit einem Ausgleichsvolumen verbunden ist und das 3-Wege-Ventil derart ausgebildet ist, dass es so geschaltet wird, dass Hydraulikfluid von der Fluidleitung mit dem niedrigeren Druck in das Ausgleichsvolumen und umgekehrt strömen kann.

Im Vergleich zu den Ausgestaltungen im Stand der Technik benötigt das erfindungsgemäße Gelenk nur wenige Bauteile, um die gewünschten hydraulischen Effekte zu erreichen. Die Trennung der Funktionen ermöglicht es, diese Ausgestaltung zu realisieren. Der Strömungswiderstand, der dem strömenden Fluid durch die hydraulischen Elemente entgegengesetzt wird, wird beim erfindungsgemäßen Gelenk im Wesentlichen durch das Drosselventil hervorgerufen. Die Verbindung der jeweiligen Fluidleitung mit dem Ausgleichsvolumen wird hingegen durch ein parallel zu dem Drosselventil wirkendes 3-Wege-Ventil hergestellt. Durch die Verwendung eines einzigen 3-Wege-Ventils, was eine bevorzugte Ausgestaltung darstellt, ist die benötigte Anzahl der Ventile und insbesondere der bewegten oder beweglichen Teile deutlich reduziert.

Dass das 3-Wege-Ventil und das Drosselventil parallel zueinander wirken bedeutet nicht zwangsläufig, dass sie im geometrischen Sinn parallel zueinander angeordnet sind oder die Strömungswege, die das Fluid beim Durchströmen des jeweiligen Ventils zurücklegen kann oder tatsächlich zurückgelegt im geometrischen Sinn parallel zueinander sein müssen. Es handelt sich vielmehr um eine parallele Wirkung wie sie beispielsweise durch die aus der elektrischen Schaltungstechnik bekannten Parallelschaltungen hervorgerufen wird.

**In** einer bevorzugten Ausgestaltung ist das 3-Wege-Ventil derart ausgebildet, dass es allein durch die Druckdifferenz zwischen den beiden Fluidleitungen geschaltet wird. **In** dieser besonders bevorzugten Ausgestaltung ist folglich keine elektrische Steuerung, also insbesondere keine elektronische Datenverarbeitungseinrichtung und kein elektronischer Sensor nötig, um den Druck in wenigstens einer der beiden Fluidleitungen, vorzugsweise in beiden Fluidleitungen oder die Druckdifferenz zwischen den beiden Fluidleitungen zu messen. Vielmehr führt die Druckdifferenz selbst dazu, dass das 3-Wege-Ventil entsprechend geschaltet wird.

Vorzugsweise verfügt das 3-Wege-Ventil dazu über ein Schaltelement, das durch die Druckdifferenz zwischen den beiden Fluidleitungen so verschoben wird, dass die Verbindung zwischen der Fluidleitung mit dem höheren Druck und dem Ausgleichsvolumen geschlossen und die Verbindung zwischen der Fluidleitung mit dem geringeren Druck und dem Ausgleichsvolumen geöffnet wird. Besonders bevorzugt weist das Schaltelement einen Stößel, eine Kugel oder einen Stift auf.

Vorteilhafterweise verfügt das Gelenk über wenigstens einen Sensor, der eingerichtet ist, eine Druckdifferenz zwischen den beiden Fluidleitungen zu messen, wobei das Gelenk eine elektrische Steuerung aufweist, die eingerichtet ist, das 3-Wege-Ventil in Abhängigkeit der vom Sensor gemessenen Druckdifferenz zu schalten. Diese Ausgestaltung ist gegenüber der zuvor beschriebenen, bei der die Schaltung durch die Druckdifferenz selbst erfolgt, aufwendiger in der Produktion und der Konstruktion. Sie hat jedoch den Vorteil, dass auch kleinere und kleinste Druckdifferenzen zwischen den beiden Fluidleitungen ausreichen können, um das 3-Wege-Ventil zu schalten. Es ist in dieser Ausgestaltung nicht notwendig, dass die zum Schalten des 3-Wege-Ventils nötige Energie und Kraft aus der Druckdifferenz entnommen oder von der Druckdifferenz aufgebracht wird. Die elektrische Steuerung beinhaltet vorzugsweise wenigstens eine elektronische
Datenverarbeitungseinrichtung.

Bevorzugt verfügt das Gelenk über zwei Sensoren, von denen jeweils einer eingerichtet ist, den Druck in einer der beiden Fluidleitungen zu messen. Jeder Sensor sendet seine Messdaten an die elektrische Steuerung, die eingerichtet ist, anhand der Messdaten das 3-Wege-Ventil zu steuern. Dies kann beispielsweise anhand einer Wertetabelle geschehen, die in einem elektronischen Datenspeicher, auf den die elektrische Steuerung zugreifen kann, hinterlegt ist. Die elektrische Steuerung ist dann, zweiter eingerichtet, die Messdaten eines Sensors, der die Druckdifferenz zwischen den beiden Fluidleitungen misst, mit hinterlegten Daten zu vergleichen und entsprechend des Ergebnisses dieses Vergleiches das 3-Wege-Ventil zu steuern. Alternativ oder zusätzlich dazu ist die elektrische Steuerung eingerichtet, aus Messdaten zweier Sensoren, die jeweils Informationen über den Druck in einer der beiden Fluidleitungen enthalten, eine Druckdifferenz zu berechnen und anschließend auf der Basis der so ermittelten Druckdifferenz das 3-Wege-Ventil zu steuern.

Alternativ oder zusätzlich dazu verfügt das Gelenk über wenigstens einen Sensor, der eingerichtet ist, eine auf wenigstens eines der Gelenkteile wirkende Kraft und/oder ein auf wenigstens eines der Gelenkteile wirkendes Moment und/oder einen Relativwinkel zwischen den beiden Gelenkteilen zu bestimmen. Alternativ oder zusätzlich dazu verfügt das Gelenk für wenigstens einen Inertialsensor, der eingerichtet ist, einen Absolutwinkel, eine Lage und/oder eine Orientierung eines der Gelenkteile im Raum zu bestimmen. Besonders bevorzugt verfügt das Gelenk über wenigstens zwei derartige Inertialsensoren, von denen wenigstens einer eingerichtet ist, die entsprechende Größe des ersten Gelenkteiles zu bestimmen und wenigstens einer eingerichtet ist, die entsprechende Größe des zweiten Gelenkteiles zu bestimmen. Vorzugweise werden zur Steuerung des 3-Wege-Ventils Messdaten wenigstens eines Sensors verwendet, die auch zur Steuerung des wenigstens einen Drosselventils verwendet werden.

Vorzugsweise ist die elektrische Steuerung eingerichtet, aus den Messdaten wenigstens eines Sensors eine aktuelle Gangphase zu erkennen, in der sich das Gelenk befindet, was insbesondere von Vorteil ist, wenn das Gelenk Teil einer orthopädietechnischen Einrichtung für die untere Extremität ist. Besonders bevorzugt wird das Gelenk dann als Kniegelenk einer Orthese oder Prothese eingesetzt.

Vorzugsweise wird das Ausgleichsvolumen als Energiespeicher verwendet. Dafür ist es von Vorteil, wenn das 3-Wege-Ventil verstellbar ausgebildet ist. Besonders bevorzugt wird beim Befüllen des Ausgleichsvolumens ein mechanischer Energiespeicher, beispielsweise ein elastisches Element, beispielsweise eine Feder, gespannt und mit potentieller Energie aufgeladen.

Vorzugsweise verfügt das Gelenk über eine elektrische Steuerung, die eingerichtet ist, den durch das Drosselventil hervorgerufenen Strömungswiderstand einzustellen. Besonders bevorzugt handelt sich bei dieser elektrischen Steuerung um die elektrische Steuerung, die auch eingerichtet ist, das 3-Wege-Ventil zu schalten.

Vorzugsweise ist die elektrische Steuerung eingerichtet, den Strömungswiderstand für unterschiedliche Strömungsrichtungen durch das Drosselventil unterschiedlich groß einzustellen. Dadurch wird es möglich, einer Bewegung des Gelenks in eine erste Richtung einen anderen Strömungswiderstand entgegenzusetzen und somit diese Bewegung anders zu dämpfen als die Bewegung des Gelenks in eine zweite Richtung, die der ersten Richtung entgegengesetzt ist.

Bevorzugt ist das Drosselventil so angeordnet, dass es bei einer Verschwenkung des ersten Gelenkteils relativ zu dem zweiten Gelenkteil in unterschiedliche Schwenkrichtungen aus unterschiedlichen Strömungsrichtungen angeströmt wird.

Vorzugsweise verfügt das 3-Wege-Ventil über ein Vorspannelement, beispielsweise eine Feder, dass das Ventil in eine Schaltposition vorspannt. **In** der Schaltposition ist aber vorzugsweise die Verbindung zwischen der ersten Fluidleitung und dem Ausgleichsvolumen oder der zweiten Fluidleitung und dem Ausgleichsvolumen geöffnet. Eine solche Vorspannung hat zur Folge, dass das Schalten des 3-Wege-Ventils in die Schaltposition weniger Kraft erfordert, als aus der Schaltposition heraus. Dies ist insbesondere dann interessant, wenn der Schaltvorgang durch die Druckdifferenz zwischen den beiden Fluidleitungen hervorgerufen wird, weil durch die Vorspannung für das Schalten des 3-Wege-Ventils in die Schaltposition hinein eine geringere Druckdifferenz ausreicht als für das Schalten des 3-Wege-Ventils aus der Schaltposition heraus.

Alternativ dazu wird das 3-Wege-Ventil durch das wenigstens eine Vorspannelement in eine neutrale Stellung vorgespannt, in der keine der beiden Fluidleitungen vollständig geschlossen ist. Erst durch eine Druckdifferenz zwischen den beiden Fluidleitungen wird das 3-Wege-Ventil in eine Schaltposition gebracht. Ist keine Druckdifferenz vorhanden oder ist die vorhandene Druckdifferenz kleiner ein vorbestimmter Grenzwert, verbleibt das 3-Wege-Ventil in der neutralen Stellung und Fluid aus beiden Hydraulikkammern kann durch die teilweise geöffneten Ausgänge des 3-Wege-Ventils in das Ausgleichsvolumen oder aus ihm herausströmen. Dies ist insbesondere dann von Vorteil, wenn Volumenschwankungen beispielsweise aufgrund von Temperaturänderungen in beiden Hydraulikkammern auftreten.

Mit Hilfe der beigefügten Zeichnungen werden nachfolgend einige Ausführungsbeispiele der vorliegenden Erfindung näher erläutert. Es zeigt:
- Figur 1: - die schematische Darstellung eines Hydrauliksystems in einer ersten Situation,
- Figur 2: - die Darstellung des Hydrauliksystems in einer zweiten Situation,
- Figur 3: - die schematische Darstellung eines 3-Wege-Ventils und
- Figur 4: - die schematische Darstellung eines 3-Wege-Ventils in einer weiteren Ausführungsform.

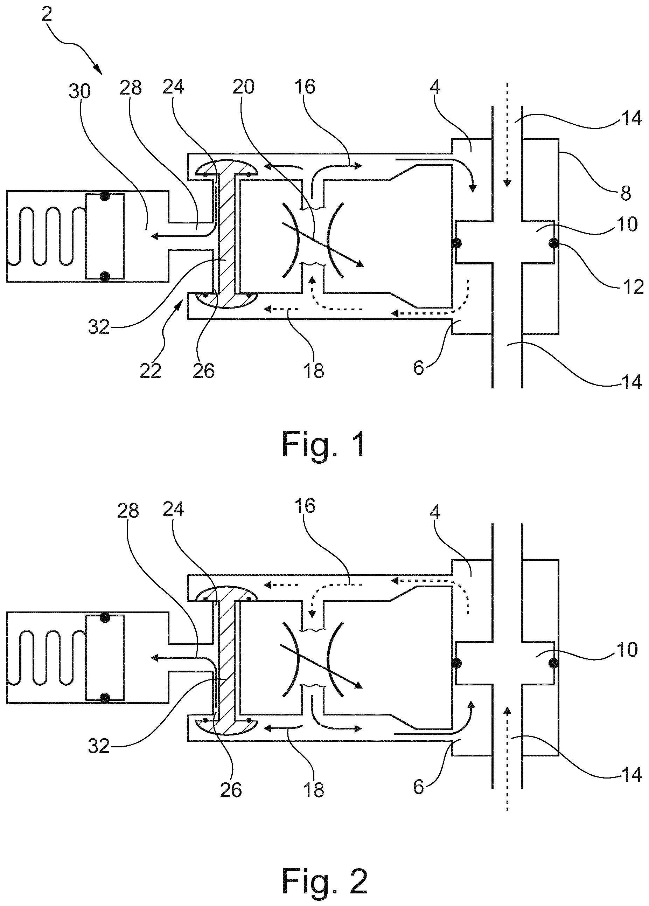

Figur 1 zeigt ein Hydrauliksystem 2 für eine orthopädietechnische Einrichtung gemäß einem Ausführungsbeispiel der vorliegenden Erfindung. Es verfügt über eine erste Hydraulikkammer 4 und eine zweite Hydraulikkammer 6, die im gezeigten Ausführungsbeispiel in einem gemeinsamen Gehäuse 8 angeordnet und durch einen Kolben 10 getrennt sind. Der Kolben 10 ist innerhalb des Gehäuses 8 bewegbar angeordnet. Am Kolben befindet sich ein Dichtungselement 12, das beispielsweise in Form eines Rings aus einem elastischen Material, beispielsweise Gummi, ausgebildet sein kann und die erste Hydraulikkammer 4 und die zweite Hydraulikkammer 6 abdichtet. Am Kolben 10 befinden sich zwei Kolbenstangen 14, von denen in der gezeigten Darstellung die erste nach oben und die zweite nach unten aus dem Gehäuse 8 herausragen. Wird ein nicht dargestelltes erstes Gelenkteil gegen ein ebenfalls nicht dargestelltes zweites Gelenkteil verschoben, wird auch wird der Kolben 10 relativ zum Gehäuse 8 verschoben, wodurch eine der beiden Hydraulikkammern 4, 6 vergrößert und die jeweils andere Hydraulikkammer 6, 4 verkleinert wird.

Die erste Hydraulikkammer 4 verfügt über eine erste Fluidleitung 16. Die zweite Hydraulikkammer 6 verfügt über die zweite Fluidleitung 18. Die erste Fluidleitung 16 und die zweite Fluidleitung 18 sind über ein Drosselventil 20 miteinander verbunden. Zusätzlich sind die erste Fluidleitung 16 und die zweite Fluidleitung 18 über einen 3-Wege-Ventil 22 miteinander verbunden. Ein erster Ausgang 24 des 3-Wege-Ventils 22 ist mit der ersten Fluidleitung 16 und ein zweiter Ausgang 26 ist mit der zweiten Fluidleitung 18 verbunden. Ein dritter Ausgang 28 des 3-Wege-Ventils 22 ist mit einem Ausgleichsvolumen 30 verbunden.

In Figur 1 ist dargestellt, wie eine durch den roten Pfeil innerhalb der oberen Kolbenstange 14 dargestellte Kraft auf die Kolbenstange 14 und damit auf den Kolben 10 wirkt. Dieser wird dadurch nach unten bewegt, wodurch die zweite Hydraulikkammer 6 verkleinert und die erste Hydraulikkammer 4 vergrößert wird. Das Hydraulikfluid, das sich innerhalb des Hydrauliksystems 2 befindet, wird dadurch aus der zweiten Hydraulikkammer 6 in die zweite Fluidleitung 18 und von dort durch das Drosselventil 20 in die erste Fluidleitung 16 und die erste Hydraulikkammer 4 geleitet. Der durch das Drosselventil 20 entgegengesetzte Strömungswiderstand ist über das Drosselventil 20 einstellbar.

Innerhalb des 3-Wege-Ventils 22 befindet sich ein Schaltelement 32, das im gezeigten Ausführungsbeispiel als von oben nach unten verlaufender Stift mit jeweils einem tellerförmigen Ende ausgebildet ist. Da sich der Kolben 10 nach unten bewegt, erhöht sich in der zweiten Hydraulikkammer 6 und der zweiten Fluidleitung 18 der Druck. Dieser wirkt auch auf das untere tellerförmige Ende des Schaltelementes 32, das dadurch nach oben bewegt wird. **In** der ersten Fluidleitung 16 entsteht durch die Bewegung des Kolbens 10 ein Unterdruck, sodass das Schaltelement 32 durch die Druckdifferenz zwischen der ersten Fluidleitung 16 und der zweiten Fluidleitung 18 bewegt wird. **In** Figur 1 wird dadurch der zweite Ausgang 26 durch das untere tellerförmige Ende des Schaltelements 32 verschlossen. Gleichzeitig hat der erste Ausgang 24 geöffnet. Das Hydraulikfluid kann folglich aus der ersten Fluidleitung 16 durch den ersten Ausgang 24 des 3-Wege-Ventils 22 in das Ausgleichsvolumen 30 strömen.

Das Ausgleichsvolumen 30 verfügt vorzugsweise über ein elastisches Element, beispielsweise einen Federelement, das dafür sorgt, dass Fluid aus dem Ausgleichsvolumen 30 durch den geöffneten ersten Ausgang 24 strömt, falls der Druck in der ersten Fluidleitung 16 und der ersten Hydraulikkammer 4 kleiner ist als der Druck im Ausgleichsvolumen 30. Auf diese Weise können Volumenschwankungen beispielsweise aufgrund von Fluidverlust oder Temperaturschwankungen ausgeglichen werden. Ist das Schaltelement 32 weiterhin vorgespannt, sodass es in einer lastenfreien Situation, also ohne externe Krafteinwirkung auf den Kolben 10 in eine neutrale Stellung übergeht, in der weder der erster Ausgang 24 noch der zweite Ausgang 26 vollständig verschlossen ist, kann dieser Volumenausgleich auch durch beide Ausgänge 24, 26 erfolgen.

**In** Figur 2 ist die umgekehrte Situation dargestellt. Der Kolben 10 wird durch die nun in der unteren Kolbenstange 14 durch den Pfeil dargestellte Kraft nach oben verschoben. Dadurch steigt der Druck in der ersten Hydraulikkammer 4 und der daran anschließenden ersten Fluidleitung 16 und Unterdruck in der zweiten Hydraulikkammer 6 und der zweiten Fluidleitung 18 sinkt. Das Hydraulikfluid wird folglich durch das Drosselventil 20 aus der ersten Hydraulikkammer in die zweite Hydraulikkammer geleitet. Durch die Druckdifferenz zwischen der ersten Fluidleitung 16 und der zweiten Fluidleitung 18 wird das Schaltelement 32 des 3-Wege-Ventils 22 in Figur 2 nach unten bewegt. Dadurch wird der erste Ausgang 24 des 3-Wege-Ventils 22 geschlossen und der zweite Ausgang 26 geöffnet. Auch in dieser Situation ist folglich die Fluidleitung mit dem Ausgleichsvolumen 30 verbunden, die den geringeren Druck aufweist.

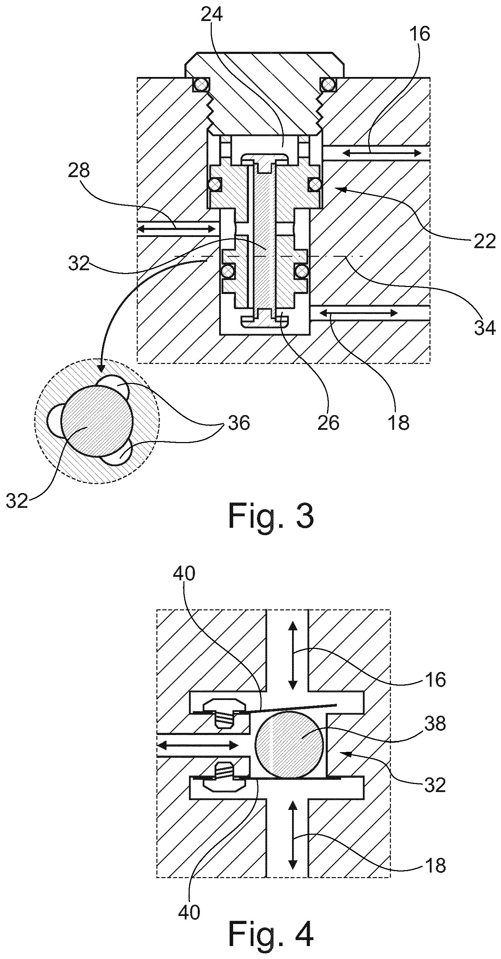

Figur 3 zeigt eine detailliertere Darstellung eines Ausschnittes eines Hydrauliksystems. Man erkennt die erste Fluidleitung 16 und die zweite Fluidleitung 18. Das 3-Wege-Ventil 22 ist in eine Ausnehmung in einem Grundkörper angeordnet. In der linken unteren Darstellung der Figur 3 ist eine Schnittdarstellung entlang der Schnittlinie 34 dargestellt. Man erkennt das Schaltelement 32, dass in der Hauptdarstellung der Figur 3 den ersten Ausgang 24 des 3-Wege-Ventils 22 schließt und den zweiten Ausgang 26 öffnet. In der Kleinschnittdarstellung unten links erkennt man das das Schaltelement 32 zwischen Kanälen 36 angeordnet ist, durch die ein Hydraulikfluid, dass durch den ersten Ausgang 24 oder den zweiten Ausgang 26 in das 3-Wege-Ventil 22 eintritt, hindurch fließen kann.

Figur 4 zeigt eine andere Ausgestaltung des 3-Wege-Ventils 22. Das Schaltelement 32 umfasst einen Schaltkörper 38, der zwischen zwei Schalthebeln 40 positioniert ist. Die Druckdifferenz zwischen der ersten Fluidleitung 16 und der zweiten Fluidleitung 18 sorgt dafür, dass unterschiedlich starke Kräfte auf die beiden Schalthebel 40 wirken, die den Schaltkörper 38 bewegen. Dadurch wird durch die Schalthebel 40 jeweils ein Ausgang des 3-Wege-Ventils 22 geöffnet und geschlossen.

### Bezugszeichenliste

- 2: Hydrauliksystem
- 4: erste Hydraulikkammer
- 6: zweite Hydraulikkammer
- 8: Gehäuse
- 10: Kolben
- 12: Dichtungselement
- 14: Kolbenstange
- 16: erste Fluidleitung
- 18: zweite Fluidleitung
- 20: Drosselventil
- 22: 3-Wege-Ventil
- 24: erster Ausgang
- 26: zweiter Ausgang
- 28: dritter Ausgang
- 30: Ausgleichsvolumen
- 32: Schaltelement
- 34: Schnittlinie
- 36: Kanal
- 38: Schaltkörper
- 40: Schalthebel

## Patentansprüche

1. Gelenk für eine orthopädietechnische Einrichtung, wobei das Gelenk
- ein erstes Gelenkteil,
- ein zweites Gelenkteil, das um eine Schwenkachse schwenkbar an dem ersten Gelenkteil angeordnet ist, und
- ein Hydrauliksystem aufweist, das
∘ eine erste Hydraulikkammer (4) mit einer ersten Fluidleitung (16) und
∘ eine zweite Hydraulikkammer (6) mit einer zweiten Fluidleitung (18) aufweist, wobei die beiden Fluidleitungen (16,18) miteinander verbunden sind,
wobei beim Verschwenken des ersten Gelenkteils relativ zum zweiten Gelenkteil Hydraulikfluid aus einer Hydraulikkammer (4,6) in die andere Hydraulikkammer (6,4) geleitet wird,
**dadurch gekennzeichnet, dass**
die Fluidleitungen (16,18) durch ein Drosselventil (20) und ein 3-Wege-Ventil (22) verbunden sind, die parallel zueinander wirken, wobei der dritte Ausgang (28) des 3-Wege-Ventils (22) mit einem Ausgleichsvolumen (30) verbunden ist und das 3-Wege-Ventil (22) derart ausgebildet ist, dass es so geschaltet wird, dass Hydraulikfluid von der Fluidleitung (16,18) mit dem niedrigeren Druck in das Ausgleichsvolumen (30) und umgekehrt strömen kann.

2. Gelenk nach Anspruch 1, **dadurch gekennzeichnet, dass** das 3-Wege-Ventil (22) derart ausgebildet ist, dass es allein durch eine Druckdifferenz zwischen den beiden Fluidleitungen (16,18) geschaltet wird.

3. Gelenk nach Anspruch 1 oder 2, **dadurch gekennzeichnet, dass** das 3-Wege-Ventil (22) ein Schaltelement (32) aufweist, das durch die Druckdifferenz zwischen den beiden Fluidleitungen (16,18) so verschoben wird, dass die Verbindung zwischen der Fluidleitung (16,18) mit dem höheren Druck und dem Ausgleichvolumen (30) geschlossen und die Verbindung zwischen der Fluidleitung (18,16) mit dem geringeren Druck und dem Ausgleichsvolumen (30) geöffnet wird.

4. Gelenk nach Anspruch 3, **dadurch gekennzeichnet, dass** das Schaltelement (32) einen Stößel, eine Kugel oder einen Stift aufweist.

5. Gelenk nach einem der vorstehenden Ansprüche, **dadurch gekennzeichnet, dass** das Gelenk über wenigstens einen Sensor verfügt, der eingerichtet ist, eine Druckdifferenz zwischen den beiden Fluidleitungen (16,18) zu messen, wobei das Gelenk eine elektrische Steuerung aufweist, die eingerichtet ist, das 3-Wege-Ventil (22) in Abhängigkeit der vom Sensor gemessenen Druckdifferenz zu schalten.

6. Gelenk nach einem der vorstehenden Ansprüche, **dadurch gekennzeichnet, dass** das Gelenk eine elektrische Steuerung aufweist, die eingerichtet ist, den durch das Drosselventil (20) hervorgerufenen Strömungswiderstand einzustellen.

7. Gelenk nach Anspruch 6, **dadurch gekennzeichnet, dass** die elektrische Steuerung eingerichtet ist, den Strömungswiderstand für unterschiedliche Strömungsrichtungen durch das Drosselventil (20) unterschiedlich groß einzustellen.

8. Gelenk nach Anspruch 6 oder 7, **dadurch gekennzeichnet, dass** das Drosselventil (20) so angeordnet ist, dass es bei einer Verschwenkung des ersten Gelenkteils relativ zum zweiten Gelenkteil in unterschiedliche Schwenkrichtungen aus verschiedenen Strömungsrichtungen angeströmt wird.

9. Gelenk nach einem der vorstehenden Ansprüche, **dadurch gekennzeichnet, dass** das 3-Wege-Ventil (22) ein Vorspannelement, beispielsweise eine Feder, besonders bevorzugt wenigstens eine Blattfeder und/oder wenigstens eine Spiralfeder, aufweist, das das 3-Wege-Ventil in eine Schaltposition vorspannt.

10. Gelenk nach einem der vorstehenden Ansprüche, **dadurch gekennzeichnet, dass** das 3-Wege-Ventil (22) ein Vorspannelement, beispielsweise eine Feder, besonders bevorzugt wenigstens eine Blattfeder und/oder wenigstens eine Spiralfeder aufweist, dass das 3-Wege-Ventil (22) in eine neutrale Position vorspannt, in der weder der erster Ausgang (24) noch der zweite Ausgang (26) vollständig verschlossen ist.

## Claims

1. A joint for an orthopedic device, the joint comprising
- a first joint part,
- a second joint part, which is arranged on the first joint part such that it can be swivelled about a swivel axis, and
- a hydraulic system which comprises
∘ a first hydraulic chamber (4) with a first fluid line (16) and
∘ a second hydraulic chamber (6) with a second fluid line (18), the two fluid lines (16,18) being connected to each other,
wherein hydraulic fluid is conducted from one hydraulic chamber (4,6) into the other hydraulic chamber (6,4) when the first joint part is swivelled relative to the second joint part,
**characterized in that**
the fluid lines (16,18) are connected by a throttle valve (20) and a 3-way valve (22) that act parallel to each other, wherein the third outlet (28) of the 3-way valve (22) is connected to a compensation volume (30) and the 3-way valve (22) is designed in such a way that it is switched so that hydraulic fluid can flow from the fluid line (16,18) with the lower pressure into the compensation volume (30) and vice-versa.

2. The joint according to claim 1, **characterized in that** the 3-way valve (22) is designed in such a way that it is switched simply by the pressure difference between the two fluid lines (16,18).

3. The joint according to claim 1 or 2, **characterized in that** the 3-way valve (22) has a switching element (32) that is displaced due to the pressure difference between the two fluid lines (16,18) in such a way that the connection between the fluid line (16,18) with the higher pressure and the compensation volume (30) is closed and the connection between the fluid line (18,16) with the lower pressure and the compensation volume (30) is opened.

4. The joint according to claim 3, **characterized in that** the switching element (32) comprises a tappet, a ball or a pin.

5. The joint according to one of the preceding claims, **characterized in that** the joint has at least one sensor that is configured to measure a pressure difference between the two fluid lines (16,18), wherein the joint comprises an electrical control unit that is configured to switch the 3-way valve (22) depending on the pressure difference measured by the sensor.

6. The joint according to one of the preceding claims, **characterized in that** the joint comprises an electrical control unit that is configured to adjust the flow resistance caused by the throttle valve (20).

7. The joint according to claim 6, **characterized in that** the electrical control unit is configured to adjust the flow resistance differently for different flow directions through the throttle valve (20).

8. The joint according to claim 6 or 7, **characterized in that** the throttle valve (20) is arranged in such a way that when the first joint part swivels relative to the second joint part in different directions, the flow is directed towards it from different flow directions.

9. The joint according to one of the preceding claims, **characterized in that** the 3-way valve (22) comprises a pre-tensioning element, such as a spring, particularly preferably at least one leaf spring and/or at least one spiral spring, which pre-loads the 3-way valve into a switching position.

10. The joint according to one of the preceding claims, characterized (22) in that the 3-way valve (22) comprises a pre-tensioning element, such as a spring, particularly preferably at least one leaf spring and/or at least one spiral spring, which pre-loads the 3-way valve (22) into a neutral position in which neither the first outlet (24) nor the second outlet (26) is completely closed.

## Revendications

1. Articulation pour un dispositif orthopédique, l'articulation comprenant
- une première partie d'articulation,
- une deuxième partie d'articulation qui est disposée sur la première partie d'articulation de manière pivotante autour d'un axe de pivotement, et
- un système hydraulique qui comprend
• une première chambre hydraulique (4) munie d'une première conduite de fluide (16) et
• une deuxième chambre hydraulique (6) munie d'une deuxième conduite de fluide (18), les deux conduites de fluide (16, 18) étant reliées entre elles,
un fluide hydraulique étant acheminé d'une chambre hydraulique (4, 6) vers l'autre chambre hydraulique (6, 4) lors du pivotement de la première partie d'articulation par rapport à la deuxième partie d'articulation,
**caractérisée en ce que**
les conduites de fluide (16, 18) sont reliées par une vanne d'étranglement (20) et par une vanne à 3 voies (22) qui agissent en parallèle l'une avec l'autre, la troisième sortie (28) de la vanne à 3 voies (22) étant reliée à un volume de compensation (30) et la vanne à 3 voies (22) étant conçue de manière à être commutée de telle sorte que le fluide hydraulique puisse s'écouler de la conduite de fluide (16, 18) ayant la pression plus faible vers le volume de compensation (30), et inversement.

2. Articulation selon la revendication 1,
**caractérisée en ce que** la vanne à 3 voies (22) est conçue de manière à être commutée par la seule différence de pression entre les deux conduites de fluide (16, 18).

3. Articulation selon la revendication 1 ou 2,
**caractérisée en ce que** la vanne à 3 voies (22) comporte un élément de commutation (32) qui est déplacé par la différence de pression entre les deux conduites de fluide (16, 18) de manière à fermer la communication entre la conduite de fluide (16, 18) ayant la pression plus élevée et le volume de compensation (30) et à ouvrir la communication entre la conduite de fluide (18, 16) ayant la pression plus faible et le volume de compensation (30).

4. Articulation selon la revendication 3,
**caractérisée en ce que** l'élément de commutation (32) comprend un poussoir, une bille ou une goupille.

5. Articulation selon l'une des revendications précédentes,
**caractérisée en ce que** l'articulation dispose d'au moins un capteur conçu pour mesurer une différence de pression entre les deux conduites de fluide (16, 18), l'articulation comprenant une commande électrique conçue pour commuter la vanne à 3 voies (22) en fonction de la différence de pression mesurée par le capteur.

6. Articulation selon l'une des revendications précédentes,
**caractérisée en ce que** l'articulation comprend une commande électrique conçue pour ajuster la résistance à l'écoulement provoquée par la vanne d'étranglement (20).

7. Articulation selon la revendication 6,
**caractérisée en ce que** la commande électrique est conçue pour ajuster différemment la résistance à l'écoulement pour différentes directions d'écoulement à travers la vanne d'étranglement (20).

8. Articulation selon la revendication 6 ou 7,
**caractérisée en ce que** la vanne d'étranglement (20) est disposée de telle sorte que, lors d'un pivotement de la première partie d'articulation par rapport à la deuxième partie d'articulation dans différentes directions de pivotement, elle est soumise à un écoulement provenant de différentes directions d'écoulement.

9. Articulation selon l'une des revendications précédentes,
**caractérisée en ce que** la vanne à 3 voies (22) comporte un élément de précontrainte, par exemple un ressort, de préférence au moins un ressort à lame et/ou au moins un ressort spiral, qui précontraint la vanne à 3 voies vers une position de commutation.

10. Articulation selon l'une des revendications précédentes,
**caractérisée en ce que** la vanne à 3 voies (22) comporte un élément de précontrainte, par exemple un ressort, de préférence au moins un ressort à lame et/ou au moins un ressort spiral, qui précontraint la vanne à 3 voies (22) vers une position neutre dans laquelle ni la première sortie (24) ni la deuxième sortie (26) ne sont complètement fermées.
